(19) Europäisches Patentamt
European Patent Office
Office européen des brevets

(11) EP 1 482 901 B1

(12) **EUROPÄISCHE PATENTSCHRIFT**

(45) Veröffentlichungstag und Bekanntmachung des Hinweises auf die Patenterteilung:
**06.09.2006 Patentblatt 2006/36**

(21) Anmeldenummer: **02748652.1**

(22) Anmeldetag: **09.04.2002**

(51) Int Cl.:
***A61K 8/41*** *(2006.01)* ***A61K 8/66*** *(2006.01)*
***A61Q 5/06*** *(2006.01)*

(86) Internationale Anmeldenummer:
**PCT/EP2002/003904**

(87) Internationale Veröffentlichungsnummer:
**WO 2003/053387 (03.07.2003 Gazette 2003/27)**

(54) **VERFAHREN ZUR FÄRBUNG KERATINISCHER FASERN**

METHOD FOR COLOURING KERATIN FIBRES

PROCEDE POUR LA COLORATION DE FIBRES KERATINIQUES

(84) Benannte Vertragsstaaten:
**AT BE CH CY DE DK ES FI FR GB GR IE IT LI LU MC NL PT SE TR**

(30) Priorität: **20.12.2001 DE 10162640**

(43) Veröffentlichungstag der Anmeldung:
**08.12.2004 Patentblatt 2004/50**

(73) Patentinhaber: **Henkel Kommanditgesellschaft auf Aktien**
**40589 Düsseldorf (DE)**

(72) Erfinder:
- **KLEEN, Astrid**
**40699 Erkrath (DE)**
- **SÄTTLER, Andrea**
**40225 Düsseldorf (DE)**
- **HÖFFKES, Horst**
**40595 Düsseldorf (DE)**
- **OTTO, Ralf**
**88422 Oggelshausen (DE)**
- **ROSE, David**
**40723 Hilden (DE)**
- **NAUMANN, Frank**
**40219 Düsseldorf (DE)**

(56) Entgegenhaltungen:
**WO-A-01/72271** **DE-A- 19 945 486**
**US-A- 5 490 980**

- **GARDNER J M ET AL: "INVESTIGATION INTO THE ACTION OF TRANSGLUTAMINASE ON HUMAN HAIR" JOURNAL OF THE SOCIETY COSMETIC CHEMISTS, NEW-YORK, NY, US, Bd. 46, Nr. 1, Februar 1995 (1995-02), Seiten 11-28, XP000929527**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 482 901 B1

Printed by Jouve, 75001 PARIS (FR)

## Beschreibung

Die Erfindung betrifft ein Verfahren zum Färben keratinischer Fasern, bei dem spezielle Farbstoffe mit Hilfe eines Enzyms an den Fasern fixiert werden, sowie ein entsprechendes Mittel zum Färben keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen.

Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Auch hier können dann Probleme hinsichtlich der Echtheit der Färbungen auftreten.

Es hat daher nicht an Anstrengungen gefehlt, die Echtheit von Färbungen keratinischer Fasern zu verbessern. Eine Entwicklungsrichtung ist die Optimierung der Farbstoffe selbst bzw. die Synthese neuer, modifizierter Farbstoffmoleküle. Eine weitere Entwicklungsrichtung ist die Suche nach Zusätzen für die Färbemittel, um die Echtheit der Färbungen zu erhöhen. Eine bekannte Problemlösung ist, dem Färbemittel UV-Filter zuzusetzen. Diese Filtersubstanzen werden beim Färbeprozeß zusammen mit dem Farbstoff auf das Haar aufgebracht, wodurch in vielen Fällen eine deutliche Steigerung der Stabilität der Färbung gegen die Einwirkung von Tages- oder Kunstlicht erzielt wird. Außerdem erhöhen die UV-Filter, wie in der DE-A1-198 53 111 beschrieben, auch die Waschechtheit der Färbungen.

Es wurde nun überraschenderweise gefunden, dass mit einem völlig neuen enzymatischem Verfahren die Echtheitseigenschaften, insbesondere die Waschechtheit, von Färbungen keratinischer Fasern deutlich erhöht werden können.

Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluß von wäßrigen Mitteln, insbesondere tensidhaltigen Mitteln wie Shampoos, ausgesetzt wird.

Ein erster Gegenstand der vorliegenden Erfindung ist daher ein Verfahren zur Färbung keratinischer Fasern, bei dem auf die Fasern (A) mindestens ein Enzym mit Aminoacyltransferase-Aktivität (EC 2.3.2) ausgewählt aus einer der Enzymklassen EC 2.3.2.13, EC 2.3.2.6, EC 2.3.2.8 und EC 2.3.2.1, sowie (B) mindestens ein Nitrofarbstoff, der eine Substrataktivität für das Enzym aufweist, aufgebracht werden.

Unter keratinischen Fasern werden erfindungsgemäß Pelze, Wolle, Federn und insbesondere menschliche

Haare verstanden.

Enzyme werden nach der offiziellen *Enzyme Nomenclature* der IUBMB mit EC-Nummer (Enzyme Commission) benannt. Eine vollständige Liste charakterisierter Enzyme aus den Enzymklassen gemäß IUBMB ist in der Datenbank SwissProt unter http://www.expasy.ch zu finden.

Enzyme aus der Enzymklasse EC 2.3.2.1 (offizieller Name: D-glutamyltransferase) katalysieren bevorzugt die Reaktion des Aminosäureesters L- oder D-Glutamin mit einem D-Glutamyl-haltigen Protein zu einem 5-Glutamyl-D-glutamyl-Protein unter Freisetzung von Ammoniak.

Enzyme aus der Enzymklasse EC 2.3.2.6 (offizieller Name: Leucyltransferase) katalysieren bevorzugt die Reaktion von L-Leucyl-’RNA oder von Phenylalanylgruppen mit einem Protein zu einem L-Leucyl- bzw. Phenylalanyl-Protein. Bevorzugt geeignete Proteine tragen eine Aminosäure ausgewählt aus der Gruppe Arginin, Lysin und Histidin am N-Terminus.

Enzyme aus der Enzymklasse EC 2.3.2.8 (offizieller Name: Arginyltransferase) katalysieren bevorzugt die Reaktion von L-Arginyl-$^{t}$RNA mit einem Protein (in Gegenwart von Mercaptoethanol und eines einwertigen Kations) zu einem L-Arginyl-Protein. Bevorzugt geeignete Proteine tragen eine Aminosäure ausgewählt aus der Gruppe Glutamat, Aspartat und Cystein am N-Terminus.

Ein Enzym, das bevorzugt in dem erfindungsgemäßen Verfahren zum Einsatz kommt, ist Transglutaminase (offizieller Name: Protein-glutamin gamma-glutamyltransferase; EC 2.3.2.13). Dieses Enzym katalysiert bevorzugt die Reaktion des Aminosäurerestes Glutamin innerhalb eines Proteins mit einem Alkylamin zu einem N5-Alkylglutamin-Protein unter der Freisetzung von Ammoniak. Ein in der Natur bevorzugtes Alkylamin, das in dieser Reaktion eine Rolle spielt, ist die Aminosäure Lysin beziehungsweise der Aminosäurerest Lysin innerhalb eines Proteins.

Im folgenden werden die Enzyme der oben genannten erfindungsgemäßen Enzymklassen allgemein als Aminoacyltransferasen bezeichnet.

Die oben genannten Enzyme mit Aminoacyltransferaseaktivität, insbesondere prinzipiell alle Enzyme mit Transglutaminaseaktivität, eignen sich zur Ausführung der vorliegenden Erfindung. Geeignet sind beispielsweise Transglutaminasen, die aus Meerschweinchenleber, *Physarum polycephalum, Medicago sativa* oder *Bacillus subtilus* gewonnen werden oder Enzyme mit Aminoacyltransferaseaktivität, insbesondere Transglutaminaseaktivität, die durch Rekombination zugänglich sind. Besonders bevorzugt sind calciumunabhängige Transglutaminasen, wie sie beispielsweise in der EP-726 317-A2 und der EP-397 606-A1 beschrieben sind und von Ajinomoto vertrieben werden. Bevorzugt sind die Handelsprodukte Activa® WM und EB der Firma Ajinomoto, besonders bevorzugt ist Activa® WM.

Der Einsatz von Transglutaminasen in kosmetischen Formulierungen ist bereits aus der Literatur bekannt So wird beispielsweise in der US-5,490,980 ein Mittel zu Behandlung menschlicher Haut, Haare oder Nägel beschrieben, mit dem Wirkstoffe, enthaltend eine primäre Amingruppe, an die Glutaminreste der Haut, der Haare oder der Nägel mittels Transglutaminase angelagert werden. Dieser Schrift sind aber keinerlei Hinweise auf den Gegenstand der vorliegenden Erfindung und die Erhöhung der Waschechtheit von Haarfärbungen mit den speziellen Nitrofarbstoffen zu entnehmen.

Unter Nitrofarbstoff mit Substrataktiviät sind erfindungsgemäß alle Nitrogruppen-haltigen Farbstoffe zu verstehen, die mittels der erfindungsgemäßen Aminoacyltransferasen an das Haar angelagert werden können. Dies kann beispielsweise durch Vernetzung der Nitrofarbstoffe mit Substrataktivität untereinander, das heißt durch Ausbildung einer Art Hülle um das Haar erfolgen. Dies kann aber bevorzugterweise auch durch kovalente Bindungen der Nitrofarbstoffe mit Substrataktivität an die Lysin- und/oder Glutaminreste der Haare erfolgen.

Erfindungsgemäß sind Nitrofarbstoffe mit Substrataktivität der Verbindung der Formel (I)

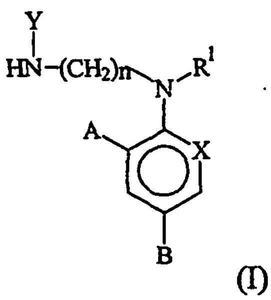

(I)

bevorzugt, wobei X steht für ein Stickstoffatom oder eine Methylengruppe, Y steht für ein Wasserstoffatom, einen L-Leucylrest, einen L-Arginylrest, einen L-Glutamylrest oder einen D-Glutamylrest, A steht für Wasserstoff, eine Nitrogruppe oder eine Trifluormethylgruppe, B steht für Wasserstoff, eine Nitrogruppe, eine Trifluormethylgruppe, eine Aminogruppe, die gegebenenfalls mit einer $C_1$- bis $C_4$-Alkylgruppe, einer $C_1$- bis $C_4$-Monohydroxyalkylgruppe, einer $C_2$- bis $C_4$-Dihy-

droxyalkylgruppe oder einer Amino-$C_1$- bis $C_4$-alkylgruppe, substituiert sein kann, oder eine Acetamidogruppe, $R^1$ steht für Wasserstoff, eine $C_1$- bis $C_4$-Alkylgruppe oder eine Arylgruppe und n steht für eine ganze Zahl von 1 bis 8, mit der Maßgabe, dass mindestens eine der Gruppen A und B für eine Nitrogruppe steht.

Besonders bevorzugte Nitrofarbstoffe mit Substrataktivität tragen ein Wasserstoffatom als Substituenten Y, insbesondere dann, wenn das erfindungsgemäße Enzym eine Transglutaminsase ist. Ganz besonders bevorzugt sind dabei die Nitrofarbstoffe mit Substrataktivität, die aus 1-(2'-Aminoethyl)amino-2-nitro-4-triflourmethylbenzol und 1-(2'-Aminoethyl)amino-4-nitrobenzol ausgewählt sind.

Die Nitrofarbstoffe mit Substrataktivität sind in den erfindungsgemäß verwendeten Mitteln bevorzugt in Mengen von 0,005 bis 10 Gew.-%, bezogen auf das gesamte Mittel, enthalten. Mengen von 0,01 bis 2 Gew.-% sind besonders bevorzugt. Das Massenverhältnis des Enzyms vom Typ der Aminoacyltransferase, insbesondere vom Typ der Transglutaminase, zum Nitrofarbstoff mit Substrataktivität beträgt bevorzugterweise 1:4000 bis 1:1, besonders bevorzugt ist ein Massenverhältnis von 1:2000 bis 1:50.

Hinsichtlich des zeitlichen Ablaufs des Färbeverfahrens unterliegt die Erfindung keinerlei Beschränkungen. Neben der Möglichkeit beide Komponenten (Enzym vom Typ der Aminoacyltransferase und Nitrofarbstoff mit Substrataktivität) gleichzeitig auf das Haar aufzubringen, ist es prinzipiell möglich, zwei separate Zubereitungen, enthaltend (a) das Enzym vom Typ der Aminoacyltransferase und (b) den Nitrofarbstoff mit Substrataktivität nacheinander in beliebiger Reihenfolge auf die Fasern aufzubringen. In einer bevorzugten Ausführungsform wird die Komponente (a) als Nachbehandlung zu der eigentlichen Färbung auf die Fasern aufgetragen.

Die Enzymzubereitung wird bevorzugterweise nach der eigentlichen Haarfärbung auf das noch feuchte Haar aufgebracht. Obwohl die Zubereitung prinzipiell auf dem Haar verbleiben kann, wird in einer bevorzugten Ausführungsform des erfindungsgemäßen Verfahrens die Zubereitung, die das Enzym enthält, nach einer Einwirkzeit von 3 bis 120 Minuten ausgespült. Dieses Ausspülen kann mit reinem Wasser erfolgen. Einwirkzeiten von 15 bis 30 Minuten haben sich in den meisten Fällen als ausreichend erwiesen.

Unabhängig von dem Ablauf des Färbeverfahrens hat es sich als vorteilhaft erwiesen, die Enzymzubereitung bei einer Temperatur von 20 bis 55 °C, insbesondere von 35 bis 50°C, anzuwenden.

In einer weiteren Ausführungsform der vorliegenden Erfindung werden neben den Nitrofarbstoffen weitere Wirkstoffe mit Substrataktivität eingesetzt. Bevorzugte Wirkstoffe mit Substrataktivität sind einerseits spezielle natürlich vorkommende Substanzen. Besonders geeignet für diese Zwecke sind Proteine, Proteinhydrolysate und deren Derivate. Proteinhydrolysate sind Produktgemische, die durch sauer, basisch oder enzymatisch katalysierten Abbau von Proteinen (Eiweißen) erhalten werden.

Erfindungsgemäß können Proteine und Proteinhydrolysate sowohl pflanzlichen als auch tierischen Ursprungs eingesetzt werden.

Tierische Proteine sind beispielsweise Elastin-, Kollagen-, Keratin-, Seiden- und Milcheiweiß-Protein. Beispiele für Proteine pflanzlichen Ursprungs sind Soja-, Mandel-, Erbsen-, Algen-, Kartoffel- und Weizenprotein.

Wenngleich der Einsatz der Proteine als solche bevorzugt ist, können an deren Stelle gegebenenfalls auch anderweitige natürliche Wirkstoffe mit Substrataktivität, wie beispielsweise Peptide, Aminosäuren und entsprechende Derivate, eingesetzt werden. Ebenfalls möglich, wenngleich weniger bevorzugt, ist der Einsatz von Derivaten der Proteinhydrolysate, beispielsweise in Form ihrer Fettsäure-Kondensationsprodukte oder kationischen Derivate.

Besonders bevorzugt sind Casein, Sojaprotein, Weizenprotein und die in der deutschen Offenlegungsschrift DE-OS-22 15 303 - auf die hier diesbezüglich explizit Bezug genommen wird - genannten Proteine und Polypeptide mit einem hohen Argininгehalt. Ganz besonders bevorzugt ist Casein.

Andererseits können im Rahmen der vorliegenden Erfindung als Wirkstoffe mit Substrataktivität auch Substanzen eingesetzt werden, die auf synthetischem Wege mit einer $H_2N$-R-Gruppe oder einer $H_2N$-(CO)-R'-Gruppe funktionalisiert sind, wobei R und R' für eine unverzweigte $C_1$- bis $C_8$-Alkylengruppe stehen. Besonders bevorzugte funktionelle Gruppen sind die von Lysin beziehungsweise Glutamin abgeleiteten Gruppen $H_2N-(CH_2)_4-$ und $H_2N-(CO)-CH_2-CH_2-$.

Weiterhin können erfindungsgemäß auch Monomere, wie beispielsweise Lysin und Glutamin, als Wirkstoffe mit Substrataktivität angeboten werden. Diese können sowohl als zusätzlicher Wirkstoff mit Substrataktivität als auch als alleinige Komponente eingesetzt werden. Es kann erfindungsgemäß bevorzugt sein, im Rahmen des Verfahrens zur Verbesserung der Waschechtheit sowohl Proteine als auch entsprechende Monomere einzusetzen, um einen schnelleren Aufbau eines dichten Netzes der Wirkstoffe mit Substrataktivität zu ermöglichen.

Die Art der Zubereitung der Enzymzubereitung unterliegt keinen prinzipiellen Einschränkungen. Erfindungsgemäß geeignet sind insbesondere wäßrige, alkoholische und ölige Zubereitungen sowie deren Mischungen. Besonders bevorzugt sind wäßrige Zubereitungen. Es kann sich beispielsweise um Lösungen, Dispersionen, Emulsionen (Wasser in Öl-Emulsionen, Öl in Wasser-Emulsionen sowie multiple Emulsionen und PIT-Emulsionen) handeln. Der pH-Wert dieser Zubereitungen liegt in der Regel bei 2 bis 10, bevorzugt bei 4 bis 9 und besonders bevorzugt bei 6 bis 8.

In einer bevorzugten Ausführungsform der vorliegenden Erfindung werden die Enzymzubereitungen in Form einer verdickten Lösung formuliert. Zu diesem Zweck werden die Mittel mit Verdickungsmitteln wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol oder auch Poylacrylsäurepolymere angedickt. Bevorzugt werden die Enzymzubereitungen niedrigviskos formuliert.

Die im Rahmen des erfindungsgemäßen Verfahrens eingesetzten Zubereitungen können außer dem Enzym und/oder dem Nitrofarbstoff mit Substrataktivität alle üblichen Bestandteile enthalten, die für die Behandlung keratinischer Fasern, insbesondere menschlicher Haare, geeignet sind. Bevorzugt sind wäßrige Zubereitungen. Unter wäßrigen Zubereitungen werden im Rahmen der Erfindungen solche Mittel verstanden, die mindestens 50 Gew.-% Wasser, bezogen auf das gesamte Mittel, enthalten.

Es hat sich als vorteilhaft erwiesen, wenn die Zubereitungen mindestens ein Tensid enthalten. Es kann sich dabei sowohl um anionische, ampholytische, zwitterionische oder nichtionogene Tenside als auch um kationische Tenside handeln. Der Fachmann kann einen eventuellen Einfluß der verschiedenen Tenside auf die Aktivität des Enzyms vom Typ der Aminoacyltransferase gegebenenfalls durch einfache Vorversuche überprüfen.

In einer bevorzugten Ausführungsform der vorliegenden Erfindung wird eine Kombination aus anionischen und nichtionischen Tensiden oder eine Kombination aus anionischen und amphoteren Tensiden eingesetzt.

Es hat sich aber in Einzelfällen als vorteilhaft erwiesen, die Tenside aus amphoteren oder nichtionischen Tensiden auszuwählen, da diese in der Regel den erfindungsgemäßen Färbeprozeß weniger beeinflussen.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel

$$R^1O\text{-}(Z)_x.$$

Diese Verbindungen sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus Cg- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3$-Gruppe tragen. Besonders geeignete zwitterionische Tenside sind die sogenannten Betaine wie die N-Alkyl-N,N-dimethylammonium-glycinate, beispielsweise das Kokosalkyldimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex® , Dehyquart® und Armocare® vertrieben. Die Produkte Armocare® VGH-70, ein N,N-Bis(2-Palmitoyloxy-ethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin enthalten die erfindungsgemäß verwendeten Zubereitungen bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0,1 Gew.-% betägt. Der Schmelzpunkt der einzelnen Öl-oder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylallcohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykol-di-isostearat, Propylenglykol-di-pelargonat, Butandiol-di-isostearat und Neopentylglykoldi-capylat erfindungsgemäß verwendbare Ölkomponenten dar, ebenso komplexe Ester wie z. B. das Diacetylglycerinmonostearat.

Schließlich können auch Fettalkohole mit 8 bis 22 C-Atomen als erfindungsgemäß wirkende Ölkomponenten eingesetzt werden. Die Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Die erfindungsgemäß verwendeten Zubereitungen enthalten gemäß einer bevorzugten Ausführungsform einen Pflegestoff. Dieser Pflegestoff ist bevorzugt ausgewählt aus kationischen Polymeren und Silikonen.

Eine erste Gruppe von kationischen Polymeren sind die sogenannten "temporär kationischen" Polymere. Diese Polymere enthalten üblicherweise eine Aminogruppe, die bei bestimmten pH-Werten als quartäre Ammoniumgruppe

und somit kationisch vorliegt.

Unter den kationischen Polymeren sind aber die permanent kationischen Polymere bevorzugt. Als "permanent kationisch" werden erfindungsgemäß solche Polymeren bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten.
Bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylaminomodifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80),
- Kationische Guar-Derivate, wie insbesondere die unter den Handelsnamen Cosmedia® Guar und Jaguar® vertriebenen Produkte,
- Polymere Dimethyldiallylammoniumsalze und deren Copolymere mit Estern und Amiden von Acrylsäure und Methacrylsäure. Die unter den Bezeichnungen Merquat® 100 (Poly(dimethyldiallylammoniumchlorid)) und Merquat® 550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethyhmethacrylat-Copolymere. Solche Verbindungen sind unter den Bezeichnungen Gafquat® 734 und Gafquat® 755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,
- sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), Polyquaternium-32, Polyquaternium-35 und Polyquaternium-37 (Handelsprodukte z. B. Salcare® SC 92 und Salcare® SC 95) bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat® ASCP 1011, Gafquat® HS 110, Luviquat® 8155 und Luviquat® MS 370 erhältlich sind.

Erfindungsgemäß bevorzugte kationische Polymere sind quaternisierte Cellulose-Derivate, polymere Dimethyldiallylammoniumsalze, Polyquaternium-27 und deren Copolymere sowie Polymere vom Typ Polyquaternium-2. Kationische Cellulose-Derivate, insbesondere das Handelsprodukt Polymer® JR 400, und Polymere vom Typ Polyquaternium-2, insbesondere das Handelsprodukt Mirapol® A-15, sind ganz besonders bevorzugte kationische Polymere.

Die kationischen Polymeren sind in den erfindungsgemäß verwendeten Mitteln bevorzugt in Mengen von 0,05 bis 10 Gew.%, bezogen auf das gesamte Mittel, enthalten. Mengen von 0,1 bis 5 Gew.% sind besonders bevorzugt.

Geeignet als Pflegestoff in Kombination mit oder alternativ zu kationischen Polymeren sind auch Ampho-Polymere. Unter dem Oberbegriff Ampho-Polymere sind amphotere Polymere, d. h. Polymere, die im Molekül sowohl freie Aminogruppen als auch freie - COOH- oder $SO_3H$-Gruppen enthalten und zur Ausbildung innerer Salze befähigt sind, zwitterionische Polymere, die im Molekül quartäre Ammoniumgruppen und -COO-- oder -$SO_3$-Gruppen enthalten, und solche Polymeren zusammengefaßt, die -COOH- oder $SO_3H$-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacrylsäure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat® 2001 N im Handel erhältlich sind, sind erfindungsgemäß besonders bevorzugte Ampho-Polymere.

Erfindungsgemäß verwendbare Pflegestoffe sind weiterhin Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie die Handelsprodukte DC 344 und DC 345 von Dow Corning, Q2-7224 (Hersteller: Dow Corning; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80) und das Handelsprodukt Fancorsil® LIM-1. Ein geeignetes anionisches Silikonöl ist das Produkt Dow Corning® 1784.

Neben den erfindungswesentlichen Komponenten und den weiteren, oben genannten bevorzugten Komponenten können die Enzymzubereitungen prinzipiell alle weiteren, dem Fachmann für solche kosmetischen Mittel bekannten Komponenten enthalten.

Weitere Wirk-, Hilfs- und Zusatzstoffe sind beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- Strukturanten wie Maleinsäure und Milchsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, $N_2O$, Dimethylether, $CO_2$ und Luft.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Obwohl der erfindungsgemäße Effekt prinzipiell bei Haaren jeglicher Art beobachtet werden kann, hat sich gezeigt, dass es besonders bevorzugt sein kann, das erfindungsgemäße Verfahren auf vorbehandelten Haaren anzuwenden. Im Rahmen einer Modellvorstellung, die den Gegenstand der Erfindung nicht einschränken soll, wird angenommen, dass die Anlagerung der Nitrofarbstoffe mit Substrataktivität verbessert werden kann, wenn die Oberfläche der Haare durch die Vorbehandlung mehr freiliegende funktionelle Gruppen aufweist.

Diese Vorbehandlung kann gemäß einer ersten Ausführungsform des Gegenstandes der vorliegenden Erfindung ein oxidativer Prozeß sein. Als Oxidationsmittel kommen sowohl anorganische als auch organische Verbindungen in Frage.

Bevorzugte anorganische Verbindungen sind Wasserstoffperoxid, Bromate, Chlorate, Iodate, Perchlorate, Peroxodisulfate, Chlorite, Bromite, Perborate, Peroxocarbonate, Peroxodiphosphate sowie Eisen(III)-, Cer(IV)- und Ruthenium (III)-Salze. Sofern es sich bei dem Oxidationsmittel um ein Salz handelt, wird das Gegenion aus den physiologisch verträglichen Ionen ausgewählt. Bei den Kationen sind dies bevorzugt Alkalimetallionen, insbesondere Kalium und Natriumionen, Erdalkalimetallionen, insbesondere Magnesiumionen, Aluminiumionen sowie Ammonium- und Mono-, Di- und Triethanolammoniumionen. Bei den Anionen sind dies bevorzugt Sulfat-, Halogenid-, insbesondere Chlorid-, Phosphat-, Acetat-, Tartrat- und Citrationen.

Besonders bevorzugte anorganische Oxidationsmittel sind Wasserstoffperoxid, Natriumbromat, Natriumperborat, Natriumpercarbonat sowie Ammonium-, Natrium- und Kaliumperoxodisulfat. Ganz besonders bevorzugt ist Wasserstoffperoxid.

Bevorzugte organische Verbindungen sind

- Benzochinone, wie z. B. 1,4-Benzochinon, 2-Methyl-1,4-benzochinon, 2,6-Dimethyl-1,4-benzochinon, 2-Chlor-1,4-benzochinon, 2-Dimethylamino-1,4-benzochinon, 2,3-Dimethyl-1,4-benzochinon, 1,4-Naphthochinon und 1,2-Naphthochinon,
- Percarbamid,
- Anlagerungsprodukte von Wasserstoffperoxid an Melamin, Polyvinylpyrrolidon und Harnstoff,
- Flavinderivate und
- Azodicarbonamid.

Besonders bevorzugte organische Verbindungen sind Benzochinone, Percarbamid und Melaminperhydrat.

Diese Vorbehandlung kann aber auch gemäß einer zweiten Ausführungsform des Gegenstandes der vorliegenden Erfindung ein reduktiver Prozeß sein. Beispiele für geeignete Reduktionsmittel sind Thioglykolsäure, Thiomilchsäure, Thioglycerol, Mercaptopropionsäure, Natriumhydrogensulfit, Ammnoniumhydrogensulfit, Cysteamin, Dithiothreitol, Thioäpfelsäure oder $\alpha$-Mercaptoethylsulfonsäure

Bevorzugte Reduktionsmittel sind Thioglykolsäure und deren Ester, Natriumhydrogensulfit, Ammoniumhydrogensulfit und Cysteamin.

Diese Vorbehandlung kann gemäß einer dritten Ausführungsform des Gegenstandes der vorliegenden Erfindung auch durch Enzyme erfolgen. Als erfindungsgemäß besonders geeignet haben sich Protein-Disulfitisomerase, Amylase, Protease, Esterase, Pronase und Lipase erwiesen. Bevorzugte Proteasen sind die Serinproteasen, wie beispielsweise Trypsin, Chymotrypsin oder Subtilisin (beispielsweise Alcalase® , Handelsprodukt der Firma Novozymes A/S; Blap® , Handelsprodukt der Firma Henkel), und die Cysteinproteasen.

Diese Vorbehandlung kann in einem separaten Schritt unmittelbar vor dem Färbeverfahren durchgeführt werden, kann aber auch bereits längere Zeit zurückliegen. So kann beispielsweise eine Dauerwellbehandlung oder eine zurückliegende oxidative Färbung oder Aufhellung der Haare bereits den durch die Vorbehandlung gewünschten Effekt bewirken. Ebenso ist dieser Effekt auf Haaren zu beobachten, die eine Schädigung durch Umwelteinflüsse, wie beispielsweise UV-Licht, aufweisen.

Da derart vorbehandelte Fasern poröser sind, stellt sich bei den Haarfärbeverfahren des Standes der Technik häufig das Problem, dass der Farbaufzug und die Echtheitseigenschaften der erzielbaren Färbungen auf derartigen Fasern deutlich verschlechtert sind. Ein Vorteil des erfindungsgemäßen Haarfärbeverfahrens besteht daher darin, dass auf derart vorbehandelten Haaren Färbungen mit einer deutlich verbesserten Waschechtheit ermöglicht werden.

Falls die Färbung der Fasern in einem separaten Schritt erfolgt, können die erfindungsgemäßen Färbemittel können neben den Nitrofarbstoffen mit Substrataktivität noch weitere Farbstoffe und/oder Farbstoffvorprodukte enthalten. Als Farbstoff(vorprodukt)e können

- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate,

sowie Mischungen von Vertretern einer oder mehrerer dieser Gruppen eingesetzt werden.

Im Rahmen dieser Ausführungsform sind hinsichtlich des Färbevorganges alle dem Fachmann bekannten Verfahren denkbar, bei denen auf das, gegebenenfalls angefeuchtete, Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. das oben genannte Buch von Kh. Schrader, verwiesen, die das entsprechende Wissen des Fachmannes wiedergeben.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2'-hydroxy-ethyl)-p-phenylendiamin, 2-(2',5'-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethyl-amino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4'-aminophenyl)-diazacycloheptan, 1,3-Bis(N(2'-hydroxyethyl)-N(4'-aminophenylamino))-2-propanol, 4-Amino-2-(2'-hydroxyethoxy)-phenol, 1,10-Bis-(2',5'-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol.

Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind

- m-Aminophenol und dessen Derivate wie beispielsweise 5-Amino-2-methylphenol, 5-(3'-Hydroxypropylamino)-2-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethyl-amino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxy-benzol, 1-Amino-3,4-methylendioxybenzol und 1-(2'-Hydroxyethyl)-amino-3,4-methylendioxybenzol.

Besonders geeignete Kupplerkomponenten sind 1-Naphthol, 1,5-, 2,7- und 1,7-Dihydroxynaphthalin, 3-Aminophenol, 5-Amino-2-methylphenel, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise in Henna rot, Henna neutral, Henna

schwarz, Kamillenblüte, Sandelholz, schwarzem Tee, Faulbaumrinde, Salbei, Blauholz, Krappwurzel, Catechu, Sedre und Alkannawurzel enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Sowohl die Oxidationsfarbstoffvorprodukte als auch die direktziehenden Farbstoffe sind in den Färbezubereitungen bevorzugt in Mengen von 0,01 bis 20 Gew.-%, vorzugsweise 0,5 bis 5 Gew.-%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIa),

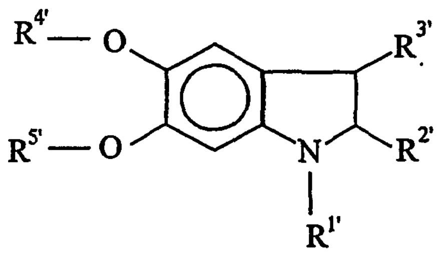

**(IIa)**

in der unabhängig voneinander

- $R^{1'}$ steht für Wasserstoff, eine $C_1$- bis $C_4$-Alkylgruppe oder eine $C_1$- bis $C_4$-Hydroxyalkylgruppe,
- $R^{2'}$ steht für Wasserstoff oder eine -COOH-Gruppe, wobei die -COOH-Gruppe auch als Salz mit einem physiologisch verträglichen Kation vorliegen kann,
- $R^{3'}$ steht für Wasserstoff oder eine $C_1$- bis $C_4$-Alkylgruppe,
- $R^{4'}$ steht für Wasserstoff, eine $C_1$- bis $C_4$-Alkylgruppe oder eine Gruppe -CO-$R^{6'}$, in der $R^{6'}$ steht für eine $C_1$- bis $C_4$-Alkylgruppe, und
- $R^{5'}$ steht für eine der unter $R^{4'}$ genannten Gruppen,

sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb),

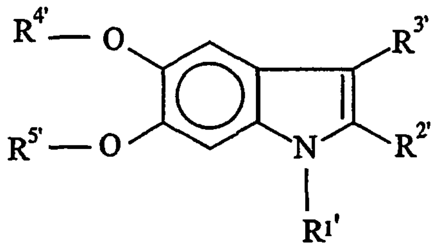

(IIb)

in der unabhängig voneinander

- $R^{1'}$ steht für Wasserstoff, eine $C_1$- bis $C_4$-Alkylgruppe oder eine $C_1$- bis $C_4$-Hydroxyalkylgruppe,
- $R^{2'}$ steht für Wasserstoff oder eine -COOH-Gruppe, wobei die -COOH-Gruppe auch als Salz mit einem physiologisch verträglichen Kation vorliegen kann,
- R3' steht für Wasserstoff oder eine $C_1$- bis $C_4$-Alkylgruppe,
- $R^{4'}$ steht für Wasserstoff, eine $C_1$- bis $C_4$-Alkylgruppe oder eine Gruppe -CO-$R^{6'}$, in der $R^{6'}$ steht für eine $C_1$- bis $C_4$-Alkylgruppe, und
- $R^{5'}$ steht für eine der unter $R^{4'}$ genannten Gruppen,

sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsaure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen. Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.%, vorzugsweise 0,2-5 Gew.% enthalten.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere $\alpha$-Aminocarbonsäuren und $\omega$-Aminocarbonsäuren. Unter den $\alpha$-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt. Weiterhin sind die Oligopeptide erfindungsgemäß bevorzugt, die mindestens eine der oben genannten Aminosäuren enthalten. Besonders bevorzugt sind Oligopeptide, die mindestens eine Arginineinheit aufweisen.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Allcalisierungsmittel, üblicherweise Alkali-oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von $\omega$-Aminosäuren wie $\omega$-Aminocapronsäure als Alkalisierungsmittel ist möglich.

Erfolgt die Ausbildung der eigentlichen Haarfarben im Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare

mit der Zubereitung der Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Ein zweiter Gegenstand der Erfindung ist die Verwendung von (A) mindestens einem Enzym vom Typ der Aminoacyltransferase, insbesondere vom Typ der Transglutaminase, und (B) mindestens einem Nitrofarbstoff, der eine Substrataktivität für das Enzym aufweist, zur Färbung keratinischer Fasern.

Ein dritter Gegenstand der vorliegenden Erfindung ist ein Mittel zum Färben keratinischer Fasern, das

(A) ein Enzym vom Typ der Aminoacyltransferase, insbesondere vom Typ der Transglutaminase, und
(B) einen Nitrofarbstoff mit Substrataktivität

enthält.

Ein vierter Gegenstand der vorliegenden Erfindung ist ein zweiteiliges Kit zum Färben keratinischer Fasern, das eine erste Zubereitung, enthaltend ein Enzym vom Typ der Aminoacyltransferase, insbesondere vom Typ der Transglutaminase, und eine zweite Zusammensetzung, enthaltend einen Nitrofarbstoff mit Substrataktivität, enthält.

Die folgenden Beispiele sollen die Erfindung näher erläutern.

## Beispiele

### Färbung

Auf Strähnen der Fa. Kerling (Humanhaar, naturweiß) wurde eine 1 Gew.-%ige Farbstofflösung, die mit einem Tris(hydroxymethyl)-aminomethan/Kaliumchlorid-Puffer-system auf einen pH-Wert von 5,5 eingestellt worden war und ferner 0,01 Gew.-% Aktivsubstanz Transglutaminase enthielt, aufgetragen und dort 30min bei Raumtemperatur belassen. Nach der Färbung wurde das Haar gespült, mit einem üblichen Haarwaschmittel ausgewaschen und anschließend getrocknet. Als Referenz wurden jeweils Strähnen mit einer Lösung behandelt, die keine Transglutaminase enthielt aber ansonsten identisch war.

### Überprüfung der Waschechtheit

Zum Nachweis des verbesserten Waschechtheit wurden die Strähnen abschließend mit einem herkömmlichen Shampoo gewaschen und getrocknet. Der Waschvorgang wurde insgesamt sechsmal durchgeführt.

Die Färbung der Strähnen wurde farbmetrisch an 4 Meßpunkten mit dem Gerät Datacolor Text Flash, der Firma Data Color International vermessen, die Meßergebnisse mit der Software Data Color Tools QC gemäß Formel (I) ausgewertet und in der folgenden Tabelle zusammengefaßt.

$$\frac{K/S_{Probe}}{K/S_{Referenz}} * 100 = \text{Farbstärke}[\%] \qquad (I)$$

mit K = Absorptionskoeffizient
S = Streukoeffizient
K/S = Reflektionskoeffizient

Zusätzlich wurde gemäß Formel (II) der ΔE-Wert des CIELAB-Farbsystems ermittelt

$$\Delta E = \sqrt{(\Delta L)^2 + (\Delta A)^2 + (\Delta B)^2} \qquad (II)$$

Je kleiner der ΔE-Wert ist, umso ähnlicher sind sich zwei Ausfärbungen.

Tabelle I: Färbungen mit einer 1 Gew.% wässrigen 1-((β-Aminoethyl)-amino)-2-nitro-4-trifluormethyl-benzol-Lösung

| | Strähnenbehandlung | ΔE | Farbstärke in % |
|---|---|---|---|
| Referenz | Ohne Transglutaminase gefärbte Strähne; vor dem Waschen | 0 | 100 |
| Blindwert | Ohne Transglutaminase gefärbte Strähne; nach dem Waschen | 3,8 | 82,8 |
| Erfindung | Mit Transglutaminase gefärbte Strähne; nach dem Waschen | 3,3 | 89,9 |

Tabelle II: Färbungen mit einer 1 Gew.% wässrigen 1-((β-Aminoethyl-amino)-4-nitrobenzol-Lösung

| | Strähnenbehandlung | ΔE | Farbstärke in % |
|---|---|---|---|
| Referenz | Ohne Transglutaminase gefärbte Strähne; vor dem Waschen | 0 | 100 |
| Blindwert | Ohne Transglutaminase gefärbte Strähne; nach dem Waschen | 2,8 | 88,7 |
| Erfindung | Mit Transglutaminase gefärbte Strähne; nach dem Waschen | 1,9 | 99,5 |

Die erfindungsgemäße gefärbten Strähnen weisen eine höhere Farbstärke und einen kleineren ΔE-Wert als die Vergleichssträhnen auf. Dies entspricht einer deutlich verbesserten Waschechtheit der erfindungsgemäß erzielten Ausfärbungen.

## Patentansprüche

**1.** Verfahren zur Färbung keratinischer Fasern, **dadurch gekennzeichnet, dass** auf die Fasern

(A) mindestens ein Enzym mit Aminoacyltransferase-Aktivität (EC 2.3.2) ausgewählt aus einer der Enzymklassen EC 2.3.2.13, EC 2.3.2.6, EC 2.3.2.8 und EC 2.3.2.1, sowie
(B) mindestens ein Nitrofarbstoff, der eine Substrataktivität für das Enzym aufweist,

aufgebracht werden.

**2.** Verfahren nach Anspruch 1, **dadurch gekennzeichnet, daß** das Enzym ein Enzym vom Typ der Transglutaminasen (EC 2.3.2.13) ist.

**3.** Verfahren nach Anspruch 1 oder 2, **dadurch gekennzeichnet, dass** das Enzym eine calciumunabhängige Transglutaminase ist.

**4.** Verfahren nach einem der Ansprüche 1 bis 3, **dadurch gekennzeichnet, dass** der Nitrofarbstoff mit Substrataktivität eine Verbindung der Formel (I)

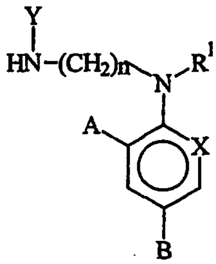

(I)

ist, wobei X steht für ein Stickstoffatom oder eine Methylengruppe, Y steht für ein Wasserstoffatom, einen L-Leucylrest, einen L-Arginylrest, einen L-Glutamykest oder einen D-Glutamylrest, A steht für Wasserstoff, eine Nitrogruppe oder eine Trifluormethylgruppe, B steht für Wasserstoff, eine Nitrogruppe, eine Trifluormethylgruppe, eine Aminogruppe, die gegebenenfalls mit einer $C_1$- bis $C_4$-Alkylgruppe, einer $C_1$- bis $C_4$-Monohydroxyalkylgruppe, einer $C_2$- bis $C_4$-Dihydroxyalkylgruppe oder einer Amino-$C_1$- bis $C_4$-alkylgruppe substituiert sein kann, oder eine Acetamidogruppe, $R^1$ steht für Wasserstoff, eine $C_1$- bis $C_4$-Alkylgruppe oder eine Arylgruppe und n steht für eine ganze Zahl von 1 bis 8, mit der Maßgabe, dass mindestens eine der Gruppen A und B für eine Nitrogruppe steht.

5. Verfahren nach einem der Ansprüche 2 oder 3, **dadurch gekennzeichnet, dass** der Nitrofarbstoff mit Substrataktivität eine Verbindung der Formel (I)

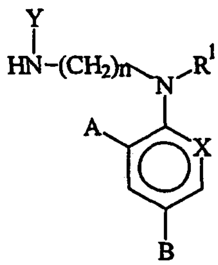

(I)

ist, wobei X steht für ein Stickstoffatom oder eine Methylengruppe, Y steht für ein Wasserstoffatom, A steht für Wasserstoff, eine Nitrogruppe oder eine Trifluormethylgruppe, B steht für Wasserstoff, eine Nitrogruppe, eine Trifluormethylgruppe, eine Aminogruppe, die gegebenenfalls mit einer $C_1$- bis $C_4$-Alkylgruppe, einer $C_1$- bis $C_4$-Monohydroxyalkylgruppe, einer $C_2$- bis $C_4$-Dihydroxyalkylgruppe oder einer Amino-$C_1$- bis $C_4$-alkylgruppe substituiert sein kann, oder eine Acetamidogruppe, $R^1$ steht für Wasserstoff, eine $C_1$- bis $C_4$-Alkylgruppe oder eine Arylgruppe und n steht für eine ganze Zahl von 1 bis 8, mit der Maßgabe, dass mindestens eine der Gruppen A und B für eine Nitrogruppe steht.

6. Verfahren nach Anspruch 5, **dadurch gekennzeichnet, dass** der Nitrofarbstoff mit Substrataktivität ausgewählt ist aus 1-(2'-Aminoethyl)amino-2-nitro-4-trifluormethylbenzol und 1-(2'-Aminoethyl)amino-4-nitrobenzol.

7. Verfahren nach einem der Ansprüche 1 bis 6, **dadurch gekennzeichnet, dass** in einem ersten Schritt der Nitrofarbstoff mit Substrataktivität und in einem zweiten Schritt die Enzymzubereitung auf die Fasern aufgebracht werden.

8. Verfahren nach Anspruch 7, **dadurch gekennzeichnet, dass** in dem im ersten Schritt eingesetzten Mittel weitere Farbstoff(vorprodukt)e eingesetzt werden.

9. Verfahren nach einem der Ansprüche 1 bis 8, **dadurch gekennzeichnet, dass** die Einwirkzeit 3 bis 120 Minuten beträgt.

10. Verwendung von (A) mindestens einem Enzym vom Typ der Aminoacyltransferase gemäß Anspruch 1, insbesondere vom Typ der Transglutaminase, und (B) mindestens einem Nitrofarbstoff, der eine Substrataktivität für das Enzym aufweist, zur Färbung keratinischer Fasern.

**11.** Mittel zum Färben keratinischer Fasern, **dadurch gekennzeichnet, dass** es

(A) ein Enzym vom Typ der Aminoacyltransferase gemäß Anspruch 1, insbesondere vom Typ der Transglutaminase, und
(B) einen Nitrofarbstoff mit Substrataktivität

enthält.

**12.** Zweiteiliges Kit zum Färben keratinischer Fasern, **dadurch gekennzeichnet, dass** es eine erste Zubereitung, enthaltend einen Nitrofarbstoff mit Substrataktivität, und eine zweite Zusammensetzung, enthaltend ein Enzym vom Typ der Aminoacyltransferase gemäß Anspruch 1, insbesondere vom Typ der Transglutaminase, enthält

## Claims

**1.** A process for coloring keratin fibers, **characterized in that**

(A) at least one enzyme with aminoacyltransferase activity (EC 2.3.2) selected from one of the enzyme classes EC 2.3.2.13, EC 2.3.2.6, EC 2.3.2.8 and EC 2.3.2.1 and
(B) at least one nitro dye with substrate activity for the enzyme

are applied to the fibers.

**2.** A process as claimed in claim 1, **characterized in that** the enzyme is an enzyme of the transglutaminase type (EC 2.3.2.13).

**3.** A process as claimed in claim 1 or 2, **characterized in that** the enzyme is a calcium-independent transglutaminase.

**4.** A process as claimed in any of claims 1 to 3, **characterized in that** the nitro dye with substrate activity is a compound corresponding to formula (I):

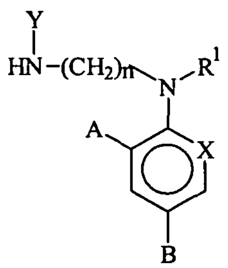

(I)

where X is a nitrogen atom or a methylene group, Y is a hydrogen atom, an L-leucyl group, an L-arginyl group, an L-glutamyl group or a D-glutamyl group, A is hydrogen, a nitro group or a trifluoromethyl group, B is hydrogen, a nitro group, a trifluoromethyl group, an amino group, which may optionally be substituted by a $C_{1-4}$ alkyl group, a $C_{1-4}$ monohydroxyalkyl group, a $C_{2-4}$ dihydroxyalkyl group or an amino-$C_{1-4}$-alkyl group, or an acetamido group, $R^1$ is hydrogen, a $C_{1-4}$ alkyl group or an aryl group and n is an integer of 1 to 8, with the proviso that at least one of the groups A and B is a nitro group.

**5.** A process as claimed in claim 2 or 3, **characterized in that** the nitro dye with substrate activity is a compound corresponding to formula (I):

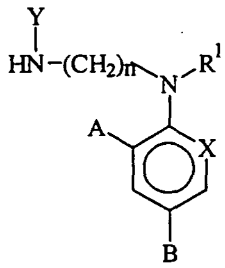

(I)

where X is a nitrogen atom or a methylene group, Y is a hydrogen atom, A is hydrogen, a nitro group or a trifluoromethyl group, B is hydrogen, a nitro group, a trifluoromethyl group, an amino group, which may optionally be substituted by a $C_{1-4}$ alkyl group, a $C_{1-4}$ monohydroxyalkyl group, a $C_{2-4}$ dihydroxyalkyl group or an amino-$C_{1-4}$-alkyl group, or an acetamido group, $R^1$ is hydrogen, a $C_{1-4}$ alkyl group or an aryl group and n is an integer of 1 to 8, with the proviso that at least one of the groups A and B is a nitro group.

6. A process as claimed in claim 5, **characterized in that** the nitro dye with substrate activity is selected from 1-(2'-aminoethyl)-amino-2-nitro-4-trifluoromethyl benzene and 1-(2'-aminoethyl)-amino-4-nitrobenzene.

7. A process as claimed in any of claims 1 to 6, **characterized in that** the nitro dye with substrate activity and the enzyme preparation are applied to the fibers in a first step and a second step, respectively.

8. A process as claimed in claim 7, **characterized in that** other dye (precursors) are used in the composition applied in the first step.

9. A process as claimed in any of claims 1 to 8, **characterized in that** the contact time is 3 to 120 minutes.

10. The use of (A) at least one enzyme of the aminoacyltransferase type according to claim 1, more particularly of the transglutaminase type, and (B) at least nitro dye with substrate activity for the enzyme for coloring keratin fibers.

11. A composition for coloring keratin fibers, **characterized in that** it contains

(A) an enzyme of the acylaminotransferase type according to claim 1, more particularly of the transglutaminase type, and
(B) at least one nitro dye with substrate activity.

12. A two-part kit for coloring keratin fibers, **characterized in that** it contains a first preparation containing a nitro dye with substrate activity and a second composition containing an enzyme of the acylaminotransferase type according to claim 1, more particularly of the transglutaminase type.

**Revendications**

1. Procédé pour la coloration de fibres kératiniques, **caractérisé en ce qu'**on dépose sur les fibres

(A) au moins une enzyme à activité d'aminoacyl transférase (EC 2.3.2) choisie parmi l'une des classes d'enzymes EC 2.3.2.13, EC 2.3.2.6, EC 2.3.2.8 et EC 2.3.2.1, ainsi que
(B) au moins un colorant nitro qui présente une activité de substrat pour l'enzyme.

2. Procédé selon la revendication 1, **caractérisé en ce que** l'enzyme est une enzyme du type des transglutaminases (EC 2.3.2.13).

3. Procédé selon l'une des revendications 1 ou 2, **caractérisé en ce que** l'enzyme est une transglutaminase dépendant du calcium.

4. Procédé selon l'une des revendications 1 à 3, **caractérisé en ce que** le colorant nitro ayant une activité de substrat

est un composé de formule (I)

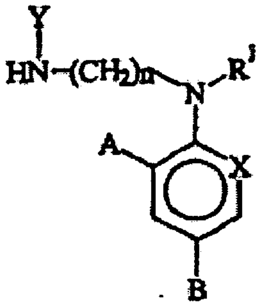

(I)

dans laquelle X représente un atome d'azote ou un groupe méthylène, Y représente un atome d'hydrogène, un radical L-leucyle, un radical L-arginyle, un radical L-glutamyle ou un radical D-glutamyle, A représente un hydrogène, un groupe nitro ou un groupe trifluorométhyle, B représente un hydrogène, un groupe nitro, un groupe trifluorométhyle, un groupe amino, qui peut le cas échéant être substitué par un groupe alkyle en $C_1$ à $C_4$, un groupe monohydroxyalkyle en $C_1$ à $C_4$, un groupe dihydroxyalkyle en $C_2$ à $C_4$ ou un groupe amino-alkyle en $C_1$ à $C_4$, ou un groupe acétamido, $R^1$ représente un hydrogène, un groupe alkyle en $C_1$ à $C_4$ ou un groupe aryle et n représente un nombre entier allant de 1 à 8, sous réserve qu'au moins un des groupes A et B représente un groupe nitro.

5. Procédé selon l'une des revendications 2 ou 3, **caractérisé en ce que** le colorant nitro ayant une activité de substrat est un composé de formule (I)

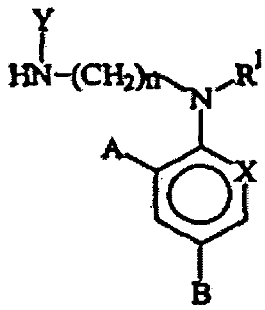

(I)

dans laquelle X représente un atome d'azote ou un groupe méthylène, Y représente un atome d'hydrogène, A représente un hydrogène, un groupe nitro ou un groupe trifluorométhyle, B représente un hydrogène, un groupe nitro, un groupe trifluorométhyle, un groupe amino qui peut être le cas échéant substitué par un groupe alkyle en $C_1$ à $C_4$, un groupe monohydroxyalkyle en $C_1$ à $C_4$, un groupe dihydroxyalkyle en $C_2$ à $C_4$ ou un groupe amino-alkyle en $C_1$ à $C_4$, ou un groupe acétamido, $R^1$ représente un hydrogène, un groupe alkyle en $C_1$ à $C_4$ ou un groupe aryle et n représente un nombre entier allant de 1 à 8, sous réserve qu'au moins un des groupes A et B représente un groupe nitro.

6. Procédé selon la revendication 5, **caractérisé en ce que** le colorant nitro ayant une activité de substrat est choisi parmi le 1-(2'-aminoéthyl)amino-2-nitro-4-trifluorométhylbenzène et le 1-(2'-aminoéthyl)amino-4-nitrobenzène.

7. Procédé selon l'une des revendications 1 à 6, **caractérisé en ce que** dans une première étape on dépose sur les fibres le colorant nitro ayant une activité de substrat et dans une seconde étape la préparation d'enzyme.

8. Procédé selon la revendication 7, **caractérisé en ce que** dans le produit utilisé dans la première étape on emploie d'autres (précurseurs de) colorants.

**9.** Procédé selon l'une des revendications 1 à 8, **caractérisé en ce que** le temps d'action est de 3 à 120 minutes.

**10.** Utilisation de (A) au moins une enzyme du type de l'aminoacyl transférase selon la revendication 1, en particulier du type de la transglutaminase, et (B) au moins un colorant nitro qui présente une activité de substrat pour l'enzyme, pour la coloration de fibres kératiniques.

**11.** Produit de coloration de fibres kératiniques, **caractérisé en ce qu'**il contient

(A) une enzyme du type de l'aminoacyl transférase selon la revendication 1, en particulier du type de la transglutaminase, et
(B) un colorant nitro ayant une activité de substrat.

**12.** Nécessaire en deux parties pour la coloration de fibres kératiniques, **caractérisé en ce qu'**il contient une première préparation contenant un colorant nitro ayant une activité de substrat, et une seconde composition contenant une enzyme du type de l'aminoacyl transférase selon la revendication 1, en particulier du type de la transglutaminase.